Europäisches Patentamt

European Patent Office

Office européen des brevets

(11) Numéro de publication: **0 273 816 B1**

# FASCICULE DE BREVET EUROPEEN

(45) Date de publication de fascicule du brevet:
**26.06.91**

(51) Int. Cl.[5]: **B01J 29/04**, C01B 33/20

(21) Numéro de dépôt: **87402779.0**

(22) Date de dépôt: **08.12.87**

(54) **Nouveau procédé de synthèse de zéolithes du type ferrisilicate, produits ainsi obtenus et leurs utilisations.**

(30) Priorité: **16.12.86 FR 8617711**

(43) Date de publication de la demande:
**06.07.88 Bulletin 88/27**

(45) Mention de la délivrance du brevet:
**26.06.91 Bulletin 91/26**

(84) Etats contractants désignés:
**AT BE DE ES GB IT NL**

(56) Documents cités:
EP-A- 0 010 572
EP-A- 0 030 751
EP-A- 0 115 031
EP-A- 0 160 136
CA-A- 1 197 498

(73) Titulaire: **INSTITUT FRANCAIS DU PETROLE**
**4, Avenue de Bois-Préau**
**F-92502 Rueil-Malmaison(FR)**

(72) Inventeur: **Patarin, Joel**
**13, rue Eugène Delacroix Appt 64**
**F-68200 Mulhouse(FR)**
Inventeur: **Guth, Jean-Louis**
**59, rue Bellevue Brunstatt**
**F-68200 Mulhouse(FR)**
Inventeur: **Kessler, Henri**
**17, rue de la Forêt**
**F-68270 Wittenheim(FR)**
Inventeur: **Coudurier, Gisèle**
**11, rue Bailli de Suffren**
**F-69330 Meyzieu(FR)**
Inventeur: **Raatz, Francis**
**17, rue Michelet**
**F-92500 Rueil Malmaison(FR)**

## Description

La présente invention concerne un nouveau procédé de synthèse de zéolithes du type ferrisilicate appelées ferrizéolites, les produits obtenus par ce procédé ainsi que leurs applications en adsorption et en catalyse.

Les ferrizéosilites sont des tectosilicates cristallisés. Leur structure tridimensionnelle est construite par un assemblage de tétraèdres $TO_4$, mettant en commun leurs sommets, deux tétraèdres différents n'ayant qu'un oxygène en commun. Dans les zéolithes du type aluminosilicate, qui sont les plus communes, T représente le silicium tétravalent ainsi que l'aluminium trivalent. Les cavités et canaux, de dimensions moléculaires, de la charpente covalente accueillent les cations compensant le déficit de charge lié à la présence de l'aluminium trivalent dans les tétraèdres. On connait aussi quelques rares zéolithes où le silicium est remplacé par du germanium tétravalent. De même, des éléments trivalents comme le gallium et plus rarement le bore ou divalents comme le beryllium peuvent se substituer à l'aluminium.

D'une manière générale, la composition des zéolithes peut être représentée par la formule brute $M_{2/n}O$ $Y_2O_3$, $xZO_2$ à l'état déshydraté et calciné. Z et Y représentent respectivement les éléments tétravalent et trivalent des tétraèdres $TO_4$ ; M représente un élément électropositif de valence n, tel qu'un alcalin ou alcalino-terreux ; x peut varier de 2 à théoriquement l'infini auquel cas la zéolithe est une silice.

Chaque type de zéolithe possède une structure poreuse distincte. La variation des dimensions et formes des pores d'un type à l'autre entraîne un changement des propriétés adsorbantes. Seules les molécules de certaines dimensions et formes sont capables d'entrer dans les pores d'une zéolithe particulière. La composition chimique avec, en particulier, la nature des cations de compensation échangeables, est également un facteur important intervenant dans la sélectivité de l'adsorption et surtout dans les propriétés catalytiques de ces produits.

Du fait de leurs caractéristiques particulières (tamis moléculaires et échangeurs cationiques), les zéolithes sont utilisées aussi bien en adsorption qu'en catalyse. Parmi les utilisations en adsorption on peut citer la purification des gaz, la séparation d'hydrocarbures ; en catalyse plusieurs procédés importants utilisent des zéolithes comme catalyseurs : craquage catalytique, hydrocraquage, isomérisation etc.

Bien que de nombreuses zéolithes du type aluminosilicate existent dans la nature, la recherche de produits ayant des propriétés nouvelles a conduit au cours de ces dernières années à la synthèse d'une grande variété de ces aluminosilicates à structure zéolithique.

Par ailleurs, plusieurs brevets revendiquent la substitution partielle voire totale des atomes d'aluminium de la charpente cristalline par des éléments de degré d'oxydation III (bore, gallium, fer...). Après remplacement des cations de compensation par des protons à partir de techniques connues dans l'art antérieur, on obtient des solides acides dont la force des sites va varier en fonction de la nature du ou des éléments (bore, gallium, fer...) ayant substitué le silicium au sein de la charpente. Ainsi par exemple des zéolithes contenant du silicium et du fer dans leur charpente cristalline ont des propriétés acides différentes de celles des zéolithes de même structure cristalline contenant du silicium et de l'aluminium dans leur charpente.

A titre d'exemple, on peut citer, dans le cas d'une substitution de Al par $Fe^{III}$, le brevet allemand DE 2831611, les brevets européens EP 813532, EP 884422 et EP 115031.

Par ailleurs, l'art antérieur peut être illustré par les brevets EP-A-160.136, EP-A-0.030.751 et CA-A-1.197.498.

De manière générale, les zéolithes sont préparées par cristallisation hydrothermale de mélanges réactionnels contenant des sources d'hydroxydes alcalins ou alcalino-terreux, de silice, et d'oxydes ou de sels d'éléments comme l'aluminium pouvant remplacer le silicium dans les tétraèdres.

L'adjonction au mélange réactionnel d'un structurant généralement organique, comme une amine ou un sel d'ammonium quaternaire, est souvent nécessaire pour l'élaboration de ladite zéolithe. Le pH de l'ensemble de la préparation est basique et généralement supérieur à 10. On admet que la concentration en ions $OH^-$ facilite la cristallisation de la zéolithe en assurant la dissolution des sources de silice, et, éventuellement des oxydes amphotères comme l'alumine, ainsi que le transfert des espèces solubles ainsi obtenues sur la zéolithe en voie de formation.

Cette méthode de synthèse des zéolithes présente de nombreux inconvénients surtout si l'on veut remplacer l'aluminium par du fer. En effet, dans un milieu basique, la plupart des zéolithes synthétisées sont métastables et on risque l'apparition, au cours de la préparation, de phases solides plus stables mais non désirées, ainsi que la précipitation d'hydroxyde ferrique. Cette difficulté ne fait que s'accroître lorsque les quantités préparées augmentent, c'est-à-dire lorsque l'on passe au stade industriel.

Par ailleurs, ces zéolithes métastables dans le milieu réactionnel basique ne sont obtenues que grâce à une forte sursaturation en espèces actives dans le milieu, ce qui provoque une nucléation rapide et, par conséquent conduit à des cristaux de zéolithe de petites tailles, les dimensions moyennes de ce cristaux se situant dans le domaine du micromètre. L'élaboration de cristaux de plus grandes tailles est donc difficile. Or, dans certaines applications d'échange d'ions, d'adsorption, ou de catalyse, il serait intéressant de pouvoir travailler avec des cristaux de grandes tailles ce qui, par exemple, permettrait d'éviter le conditionnement des zéolithes par agglomération avec tous les inconvénients que cela comporte.

De nombreuses applications, en particulier dans la catalyse acide, nécessitent des zéolithes sous une forme protonée et complètement débarrassées de leurs cations de compensation alcalins ou alcalino-terreux introduits lors de la synthèse. On peut y accéder par des procédés d'échange d'ions répétés et longs avec des cations $NH_4^+$ suivis de calcination pour les décomposer en cations $H^+$. Cette étape d'échange d'ions pourrait être éliminée si l'on pouvait remplacer entièrement les cations alcalins ou alcalino-terreux par des cations $NH_4^+$ lors de la synthèse. Or, ceci n'est pas possible lorsque le pH dépasse sensiblement 10, $NH_4^+$ étant dans ces conditions transformé en $NH_3$. Par ailleurs, les synthèses effectuées à des pH où le cation $NH_4^+$ est stable sont difficiles et longues à cause de la faible solubilité des sources de silice à ces bas pH.

L'invention a donc pour objet une zéolithe cristalline synthétique de type ferrisilicate appelée ferrizéolite appartenant à la famille des pentasils, un nouveau procédé de synthèse de cette zéolithe où les inconvénients cités précédemment sont évités et les applications de ce nouveau type de solide.

Plus précisémment la nouvelle zéolithe est caractérisée par :
a) la formule chimique approchée suivante :

$$M_{2/n}O, Fe_2O_3, xSiO_2$$

où M représente un proton résultant de la décomposition thermique des cations comme par exemple $NH_4^+$ ou tétrapropyl ou tripropylammonium ou tétrapropylphosphonium présents seuls ou en mélange dans le milieu de synthèse, et (ou) un cation de métal non décomposable issus du milieu réactionnel comme par exemple les cations alcalins et/ou alcalino-terreux ou d'autres métaux précisés ci-après,

n est la valence desdits cations

x est un nombre compris entre 40 et 1000,
b) un diagramme de diffraction X représenté dans le tableau I de la description, et
c) une teneur en fluor comprise entre environ 0,01 et 1,6 % en poids.

La zéolithe selon l'invention peut généralement présenter au moins une dimension des cristaux comprise entre 0,1 et 200 micromètres (1 $\mu$m = $10^{-6}$ m) et de préférence comprise entre 0,5 et 120 micromètres.

La ferrizéosilite de la présente invention peut généralement posséder un rapport molaire $SiO_2/Fe_2O_3$ compris entre environ 40 et 1000 et de préférence entre 50 et 750.

La présence après calcination (conditions précisées ci-après) de fluor dans la ferrizéosilite, de préférence entre 0,02 et 1,0 % poids entraîne des modifications de ses propriétés acides. Ce fluor peut si nécessaire, être éliminé par traitement dans une solution de $NH_4OH$ en autoclave entre 130 et 180° C.

Dans le cas où des cations métalliques, par exemple alcalins ou alcalino-terreux seraient introduits lors de la synthèse, ces ions peuvent être aisément éliminés par des opérations d'échanges ioniques classiques pour obtenir une ferrizéosilite acide.

L'invention concerne également les catalyseurs comprenant la nouvelle ferrizéosilite décrite ci-dessus.

La ferrizéosilite peut être en effet utilisée seule ou associée à un catalyseur. Dans ce cas le catalyseur renferme environ au moins 0,1 % et de préférence au moins 20 % de ferrizéosilite selon l'invention, le complément à 100 % étant constitué par une autre zéolithe ou par une matrice comprenant les composés choisis dans le groupe formé par l'alumine, la silice, la magnésie, la zircone, l'oxyde de titane, l'oxyde de bore, une argile et tout autre combinaison d'au moins deux des composés précités. Eventuellement le catalyseur peut comprendre au moins une fonction hydrogénante ou déshydrogénante apportée par au moins un métal choisi de préférence parmi les groupes $I_B$ et VIII ou apportée par un sulfure de métal choisi parmi les groupes $VI_B$ et VIII.

L'invention concerne aussi le procédé de préparation de ferrizéosilites du type ferrisilicate de la famille des pentasils, qui consiste en ce que :
a) On forme un mélange réactionnel en solution ayant un pH inférieur à environ 10 et comprenant de l'eau, au moins une source de silice, au moins une source de sel ferrique, au moins une source d'agent mobilisateur contenant des ions fluorure, ($F^-$) et au moins une source d'agent structurant pouvant fournir des cations organiques, par exemple les cations tétrapropylammonium ($TPA^+$), ledit mélange ayant une composition en termes de rapports molaires :

$$SiO_2/Fe_2O_3 \qquad\qquad 5\text{--}2000$$
$$F^-/SiO_2 \qquad\qquad 0,04\text{--}4$$
$$\text{Cations organiques}/SiO_2 \quad 0,04\text{--}2$$
$$H_2O/SiO_2 \qquad\qquad 6\text{--}500$$

b) On maintient ledit mélange à une température de chauffage au plus égale à 250°C jusqu'à ce qu'on obtienne un composé cristallin, et

c) On calcine ledit composé à une température supérieure à 400°C, par exemple entre 450 et 900°C.

Les sources d'agent structurant pouvant fournir des cations organiques sont de préférence des cations tétrahydrocarbylammonium trihydrocarbylammonium, tétrahydrocarbylphosphonium, hydrocarbyl étant avantageusement alkyle et de manière préférée propyle.

D'autres agents structurants ou chélatants peuvent être utilisés comme reconnu dans l'art antérieur, notamment des composés ayant des fonctions amine cétone, alcool, acide, par exemple des amino-alcools, des aminoacides, des polyalcools ou des amines tertiaires.

On peut avantageusement chauffer le mélange dans un autoclave revêtu intérieurement de polytétrafluoroéthylène (PTFE) entre environ 60°C et 210°C et de préférence entre 70°C et 190°C pendant une durée qui peut varier de 0,5 à 1100 heures selon la température de réaction, jusqu'à l'obtention d'un solide cristallisé que l'on sépare des eaux-mères par filtration et qui est ensuite lavé à l'eau distillée.

De manière avantageuse on peut préparer le mélange réactionnel à un pH compris entre 2,5 et 10 et de manière préférée entre 4 et 8.

Selon un mode préféré de préparation, les rapports molaires des constituants du mélange réactionnel peuvent être compris dans les intervalles (exprimés en rapports molaires) suivants :

$$SiO_2/Fe_2O_3 \qquad\qquad 10\text{--}1000$$
$$F^-/SiO_2 \qquad\qquad 0,1\text{--}1,5$$
$$\text{Cation organique}/SiO_2 \quad 0.08\text{--}1$$
$$H_2O/SiO_2 \qquad\qquad 15\text{--}350.$$

On peut ajouter audit mélange réactionnel au moins un sel complémentaire dans un rapport molaire sel complémentaire/$SiO_2$ compris généralement entre environ 0,1 et 4 et de préférence entre 0,2 et 0,5 et/ou au moins un germe de cristal de la zéolithe formée selon l'invention dans un rapport pondéral cristal/$SiO_2$ compris généralement entre 0,01 et 0,1 et de manière préférée entre environ 0,02 et 0,03, de telle sorte que la morphologie, la taille des cristaux ainsi que la cinétique de la réaction de cristallisation peuvent être avantageusement contrôlées.

On calcine avantageusement les cristaux de zéolithe à une température comprise entre environ 520 et 590°C sous atmosphère de gaz sec comme par exemple de l'air ou un gaz inerte, de façon à décomposer l'agent structurant présent dans les pores de la zéolithe.

Après l'étape de calcination on peut introduire dans la ferrizéosilite selon l'invention, par des méthodes d'échange d'ions bien connues dans l'art antérieur, au moins un élément du tableau périodique, dont les cations peuvent être préparés en milieu aqueux et choisis dans la famille constituée par les groupes $II_A$, $III_A$, $IV_A$, $I_B$, $II_B$, $III_B$, $IV_B$ et VIII de la classification périodique des éléments. On citera à titre d'exemple les cations alcalins, alcalino-terreux, les cations de terres-rares, $Fe^{II}$, $Fe^{III}$, $Co^{II}$, $Co^{III}$, $Ni^{II}$, $Cu^{II}$, $Zn^{II}$, $Ag^{I}$, $Pt^{II}$ etc.

Le nouveau procédé s'applique à la préparation de nouvelles zéolithes de la famille des pentasils et s'apparente aux zéolithes du type MFI, tout en se distinguant de ces dernières par des particularités des diagrammes de diffraction des rayons X et des compositions chimiques.

La demanderesse a en effet découvert que les différents inconvénients liés aux méthodes de préparation de zéolithes du type ferrisilicate en milieu basique disparaissent quand on effectue les synthèses dans des milieux aqueux avec un pH généralement inférieur à 10 et contenant des ions fluorure. Ainsi, les cations dérivés des métaux alcalins ou alcalino-terreux peuvent être remplacés par des cations $NH_4^+$ avec tous les avantages découlant de l'utilisation de ces derniers cations. La solubilisation des sources de silice et de fer est assurée par les ions fluorure qui constituent l'agent mobilisateur, et qui remplacent ainsi les ions hydroxyles des milieux basiques. Dans ces conditions on peut obtenir des cristaux de zéolithes du type ferrisilicate ayant des rapports molaires $SiO_2/Fe_2O_3$ généralement compris entre environ 40 et 1000. Lesdits cristaux ont des dimensions qui peuvent être contrôlées à partir des différents paramètres de synthèse (concentration des réactifs, agitation, température, durée), les dimensions desdits cristaux pouvant varier entre 0,05 micromètres et 500 micromètres.

Les tailles des cristaux données ci-dessus, ont été mesurées à l'aide d'un microscope électronique à haute résolution. Le catalyseur, destiné à être observé par microscopie électronique à transmission est broyé dans un mortier en agate, puis mis en suspension dans de l'éthanol par ultrasons. Une goutte de cette suspension est ensuite déposée sur une grille de cuivre recouverte d'un mince film de carbone à trou. Après un bref séchage, l'échantillon est observé par la technique dite du champ clair.

On peut travailler avantageusement en milieu agité, ce qui permet de diminuer considérablement le temps de réaction.

Le pH du milieu réactionnel, inférieur à 10, peut être obtenu soit directement à partir de l'un ou plusieurs des réactifs mis en oeuvre, soit par l'ajout d'un acide, d'une base, d'un sel acide, d'un sel basique ou d'un mélange tampon complémentaire.

De nombreuses sources de silice peuvent être utilisées. On peut citer les silices sous forme d'hydrogels, d'aérogels, de suspensions colloïdales ainsi que les silices résultant de la précipitation de solutions de silicates solubles ou de l'hydrolyse d'esters siliciques comme l'ester tétraéthylique de l'acide orthosilicique $Si(OC_2H_5)_4$ ou de complexes comme le fluorosilicate de sodium $Na_2SiF_6$ ou d'ammonium $(NH_4)_2SiF_6$.

Parmi les sels ferriques utilisés, on choisira de préférence le chlorure ferrique hydraté ou non, $FeCl_3.6H_2O$ ou $FeCl_3$, le nitrate ferrique nonahydraté $Fe(NO_3)_3.9H_2O$, le sulfate ferrique pentahydraté ainsi que le perchlorate ferrique. Par ailleurs, au lieu de partir de sources séparées de silice et d'un sel ferrique on peut également prendre des sources ou les deux éléments sont combinés comme par exemple un gel ferrisilicaté fraîchement précipité.

Les anions fluorure $F^-$ peuvent généralement être introduits sous forme de sels desdits agents structurants ou d'ammonium ou de métaux alcalins comme par exemple NaF, $NH_4F$, $NH_4HF_2$, TriPA-F, TPA-F, TPP-F, ou sous forme de composés hydrolysables pouvant libérer des anions fluorure dans l'eau comme le fluorure de silicium $SiF_4$ ou le fluorosilicate d'ammonium $(NH_4)_2 SiF_6$ ou de sodium $Na_2 SiF_6$.

Les cations $TPA^+$, $TPP^+$ ou $TriPA^+$, qui sont les agents structurants, sont ajoutés de préférence sous forme de leurs sels, par exemple les bromures, les fluorures, etc, mais on peut ajouter aussi les amines correspondantes (tripropylamine par exemple), que l'on salifie ensuite par de l'acide, fluorhydrique par exemple.

Les acides ou sels acides, les bases ou sels basiques ajoutés éventuellement en complément pour amener le pH du milieu à la valeur désirée peuvent être choisis parmi les acides courants comme par exemple HF, HCl, $HNO_3$, $H_2SO_4$, $CH_3COOH$ ou les sels acides comme par exemple $NH_4HF_2$, $KHF_2$, $NaHSO_4$, $KHSO_4$, les bases courantes comme par exemple $NaHCO_3$, $Na_2CO_3$, $CH_3COONa$, $Na_2S$, NaHS ou les mélanges tampons comme par exemple ($CH_3COOH$, $CH_3COONa$) ou ($NH_4OH$, $NH_4Cl$).

La morphologie, la taille et la cinétique de formation des cristaux de zéolithe obtenus selon le procédé de l'invention peuvent être modifiées par l'introduction dans le milieu réactionnel d'au moins un sel complémentaire choisi dans le groupe des chlorures de métaux alcalins, de sulfates de métaux alcalins et du chlorure d'ammonium. On utilise avantageusement le chlorure de sodium, de potassium et/ou d'ammonium ainsi que le sulfate de sodium. la morphologie, la taille et la cinétique de formation des cristaux de zéolithe obtenus selon le procédé de l'invention peuvent également être modifiées par l'introduction dans le milieu réactionnel de germes constitués par des cristaux (broyés ou non) apparentés à la famille de zéolithes faisant l'objet de la présente demande.

L'évolution de la composition au sein des cristaux de ferrizéosilites peut être avantageusement modulée, d'une part selon les sources de silice et du sel ferrique utilisées et d'autre part selon les rapports silice/fer engagées.

Pour des rapports $SiO_2/Fe_2O_3$ supérieur à 80, lorsque l'on part de sources de silice et de sel ferrique séparées, les cristaux obtenus présentent une hétérogénéité intracristalline avec un coeur particulièrement riche en fer (rapport Si/Fe faible) et une enveloppe riche en silicium (rapport Si/Fe élevé). L'élaboration d'un gel ferrisilicaté permet par contre d'obtenir des cristaux de ferrizéosilites dont la composition est parfaitement homogène dans l'ensemble de cristaux.

Pour certaines applications il peut s'avérer intéressant de préparer des ferrizéosilites selon l'invention ayant un coeur riche en fer donc une surface externe constituée presque entièrement de silice. Dans ces conditions la surface externe des cristaux ne contient pas de sites actifs (atomes de fer) ce qui conduit à une amélioration de la sélectivité de forme des catalyseurs puisque les réactions ne peuvent se produire que dans le réseau microporeux.

L'identification des ferrizéolites obtenues selon le procédé se fait de manière commode à partir de leur diagramme de diffraction des rayons X. Ce diagramme de diffraction peut être obtenu à l'aide d'un diffractomètre en utilisant le méthode classique des poudres avec le rayonnement K $\alpha$ du cuivre. Un étalon interne permet de déterminer précisément les valeurs des angles $2\theta$ associées aux pics de diffraction. Les différentes distances interréticulaires $d_{hkl}$, caractéristiques de l'échantillon, sont calculées à partir de la relation de BRAGG. L'estimation de l'erreur de mesure $\Delta$ ($d_{hkl}$) sur $d_{hkl}$ se calcule, en fonction de l'erreur absolue $\Delta$ ($2\theta$) affectée à la mesure de $2\theta$, par la relation de BRAGG. En présence d'un étalon interne, cette erreur est minimisée et prise couramment égale à $\pm$ 0,05$^{\circ}$. L'intensité relative I/Io affectée à chaque valeur de $d_{hkl}$ est estimée à partir de la hauteur du pic de diffraction correspondant. On utilise souvent une échelle de symboles pour caractériser cette intensité : FF = très forte, F = forte, mF = moyenne à forte, m = moyenne, mf = moyenne à faible, f = faible, ff = très faible.

Le tableau ci-après, représente le diagramme de diffraction des rayons X caractéristique des zéolithes du type ferrisilicate obtenues selon l'invention et calcinées à 550$^{\circ}$C. Dans la colonne des $d_{hkl}$ on a représenté les valeurs extrêmes que peuvent prendre les différentes équi-distances réticulaires $d_{hkl}$. La colonne "o" correspond à une ferrizéolite cristallisant dans le système orthorhombique (Si/Fe faible), la colonne "m" correspond à une ferrizéolite cristallisant dans le système monoclinique (Si/Fe élevé). Les variations observées sont essentiellement liées à la nature des cations de compensation et au rapport Si/Fe de la zéolithe. Chacune de ces valeurs doit être affectée de l'erreur de mesure $\Delta$ ($d_{hkl}$) comprise entre $\pm$ 0.07 et $\pm$ 0,002 selon la valeur de $2\theta$.

L'invention concerne également l'utilisation d'une zéolithe selon l'invention seule ou associée à une matrice aussi bien en adsorption qu'en catalyse.

En effet les ferrizéolites préparées selon l'invention sont des adsorbants sélectifs ; leurs propriétés adsorbantes sont très voisines de celles des zéolithes du type aluminosilicate.

Par ailleurs, les ferrizéolites peuvent être utilisées comme catalyseurs ou supports de catalyseurs, purs ou en mélange, pour des transformations très spécifiques de composés organiques variés. On peut citer à titre d'exemple, l'alkylation, la dismutation et la transalkylation des aromatiques, l'oligomérisation des oléfines, la conversion des alcools comme le méthanol en hydrocarbures, la conversion du propène en hydrocarbures à forte teneur en aromatiques, l'amélioration de la tenue au froid des gas oils et le craquage catalytique.

Le craquage catalytique apparaît comme une des applications importantes des ferrizéosilites selon l'invention. Pour cette application on préfère utiliser les ferrizéosilites en mélange avec un catalyseur zéolithique de craquage, à raison de 0,1 à 10 % en poids, de préférence 0,5 à 5 % en poids, de ferrizéosilite dans le mélange, le complément à 100 % étant constitué par le catalyseur zéolithique et une matrice choisie parmi celles déjà connues dans l'art antérieur. De préférence le catalyseur de craquage contient une zéolithe Y, par exemple la forme stabilisée dite ultrastable (USY), la forme Y désaluminée (rapport molaire global Si/Al compris entre 3,5 et 50), la forme ReY (échangée au moins en partie avec des cations de terres rares) contenant de 0,05 à 20 % en poids de terres rares sous forme oxyde $Re_2O_3$.

L'acidité modérée de ces férrizéosilites mise en évidence par thermodésorption programmée de $NH_3$, permet d'éviter une trop grande formation de coke, et également de diminuer les réactions de craquage, ceci ayant pour conséquence d'assurer une meilleure longévité au catalyseur.

TABLEAU I.

| $d_{hkl}$ o | $d_{hkl}$ m | I | $d_{hkl}$ o | $d_{hkl}$ m | I |
|---|---|---|---|---|---|
| 11,14 | 11,10 | FF | 3,44 | 3,45 | mf |
| 9,99 | 9,92 | FF | | 3,43 | mf |
| 9,76 | 9,76 | mF | 3,38 | 3,37 | ff |
| 9,02 | 9,00 | f | 3,353 | 3,353 | mF |
| 8,08 | 8,03 | ff | 3,321 | 3,313 | mF |
| 7,45 | 7,42 | f | 3,247 | 3,254 | f |
| 7,10 | 7,06 | ff | 3,133 | 3,131 | ff |
| 6,72 | 6,69 | f | | 3,058 | m |
| 6,38 | 6,36 | m | 3,052 | | m à mf |
| 6,03 | 5,99 | mF | | 3,036 | m |
| 5,95 | 5,93 | mF | 2,990 | 2,986 | m |
| | 5,72 | m | | 2,955 | mf |
| 5,70 | | m | 2,942 | | mf |
| | 5,68 | m | | 2,921 | ff |
| 5,57 | 5,57 | m | 2,866 | 2,864 | ff |
| 5,38 | 5,38 | ff | 2,783 | 2,782 | ff |
| | 5,33 | ff | 2,733 | 2,733 | f |
| 5,15 | 5,13 | ff | 2,685 | 2,679 | ff |
| 5,04 | 5,03 | m | 2,662 | 2,660 | ff |
| 4,99 | 4,97 | mF | | 2,613 | f |
| 4,88 | 4,88 | ff | 2,605 | | f |
| 4,62 | 4,62 | mf | | 2,589 | f |
| 4,46 | 4,40 | ff | 2,510 | 2,513 | f |
| 4,37 | 4,36 | mf | 2,488 | 2,484 | mf |
| 4,27 | 4,26 | m | 2,455 | 2,457 | ff |
| 4,09 | 4,08 | ff | 2,441 | 2,441 | ff |
| 4,01 | 4,01 | f | 2,416 | 2,413 | f |
| 3,86 | 3,85 | FF | 2,395 | 2,392 | f |
| 3,82 | 3,82 | FF | 2,325 | 2,324 | ff |
| | 3,80 | FF | 2,275 | 2,274 | ff |
| | 3,76 | F | 2,203 | 2,202 | ff |
| 3,75 | 3,74 | F | 2,011 | 2,011 | m à mf |
| 3,72 | 3,71 | F | 1,992 | 1,987 | mF |
| | 3,66 | mF | | | |
| 3,65 | 3,62 | mF | | | |
| 3,56 | 3,56 | ff | | | |
| 3,49 | 3,49 | f | | | |

"o" ferrizéolite Orthorhombique ; "m" ferrizéolite monoclinique.

Les exemples qui suivent sont destinés à illustrer l'invention.

EXEMPLE 1 : Préparation d'une ferrizéosilite de rapport molaire $SiO_2/Fe_2O_3$ égal à 84.

On prépare une solution contenant 0,376 g de chlorure ferrique hexahydraté, 1,185 g de bromure de tétrapropylammonium (TPABr) et 1,030 g de fluorure d'ammonium dans 100 g d'eau. On mélange cette solution à 3,343 g de silice pulvérulente, obtenue par pyrohydrolyse du tétrachlorure de silicium et commercialisée par la société Degussa sous le nom "Aerosil", cette dernière contient 3 % en masse d'eau environ. Par ailleurs, la teneur pondérale en aluminium est inférieure à 0,002 %. La composition molaire du mélange ramenée à une mole de silice est la suivante : 1 $SiO_2$ ; 0,025 $FeCl_3.6H_2O$ ; 0,08 TPABr ; 0,5 $NH_4F$ ; 100 $H_2O$.

Le mélange (pH = 6) est chauffé pendant 15 jours à 170°C dans un autoclave renfermant un flacon de 120 $cm^3$ en polytétrafluoroéthylène ; le pH final est de 6. Le solide obtenu après filtration et lavage à l'eau bidistillée pèse 3,62 g. La taille des cristaux de forme prismatique est de 60 x 36 micromètres. La perte de masse observée, après calcination de la zéolithe à 550°C sous un mélange de 20 % d'air dans 80 % d'azote pendant 9 heures, est de 14,2 %. L'échantillon calciné, placé dans un humidificateur (eau/$NH_4Cl$), reprend 5,8 % en poids d'eau.

L'analyse par diffraction des rayons X du produit calciné réhydraté montre qu'il s'agit d'une férrizéosilite caractérisée par le diagramme de diffraction du tableau I. L'analyse chimique de cette férrizéosilite donne une teneur en fer de 2,05 % et en élément fluor de 0,06 % en poids, et un rapport molaire $SiO_2/Fe_2O_3$, égal à 84.

EXEMPLE 2 : Préparation d'une ferrizéosilite selon l'invention à partir d'un agent structurant autre que celui utilisé dans l'exemple 1.

Cet exemple illustre la possibilité d'utiliser comme agent structurant la tripropylamine à la place du bromure de tétrapropylammonium. La composition molaire du mélange réactionnel dans ce cas est la suivante : 1 $SiO_2$ (Aerosil) ; 0,05 $FeCl_3$ ; 1 tripropylamine ; 1 HF ; 50 $H_2O$. Fraction molaire engagée : 6 %.

La tripropylamine est préalablement salifiée par l'acide fluorhydrique. Par ailleurs, 0,072 g de cristaux d'une ferrizéolite préalablement synthétisée, sont également ajoutés à l'ensemble de la préparation. Les conditions de chauffage en autoclave, et les valeurs de pH sont respectivement: durée 12 jours température : 170°C, pH initial : 5 ; pH final : 4. Le solide obtenu après lavage : 5 ; pH final : 4. Le solide obtenu après lavage et filtration est traité au dithionite de sodium afin d'extraire toutes traces d'oxyde ou d'oxy-hydroxyde ferrique présentes dans ce cas particulier. Les cristaux prismatiques ainsi obtenus ont une taille voisine de 7,5 x 2 micromètres. L'analyse chimique donne des teneurs pondérales en fer et en fluor de 1,96 et de 0,2 % respectivement. Le diagramme de diffraction du produit calciné à 550°C dans les mêmes conditions que celles de l'exemple 1 est conforme à celui du tableau I. Le rapport molaire $SiO_2/Fe_2O_3$ mesuré par analyse chimique est égal à 78.

EXEMPLE 3 : Préparation d'une ferrizéosilite selon l'invention à partir d'une autre source d'ions fluorure que celle utilisée dans l'exemple 1.

Dans cette préparation on utilise comme source d'agent fluorure, du fluorure de sodium à la place du fluorure d'ammonium, les sources de silice et de fer étant par ailleurs toujours les mêmes que dans l'exemple 1. La composition molaire du mélange ainsi employé est de : 1 $SiO_2$ ; 0,025 $FeCl_36H_2O$ ; 0,5 NaF ; 100 $H_2O$ ; 0,08 TPABr. On engage 5,5 % des proportions molaires. Le mélange réactionnel est porté, en autoclave, à une température de 170°C pendant 16 jours (le pH initial est égal à 6,1).

Le solide ainsi obtenu est placé dans une cuve à ultra-sons afin de séparer les traces de gel résiduel des cristaux.

Après sonification, lavage et filtration, on récupère 2,5 g de produit dont la composition chimique pondérale est la suivante : % Si = 38,5 ; % Fe = 1,92 ; $TPA^+$ = 10,9 ; % $F^-$ = 0,3 ; % $Na^+$ = 0,03 (rapport molaire $SiO_2/Fe_2O_3$ = 80). Les cristaux prismatiques de cette férrizéosilite sont maclés et ont une taille voisine de 60 x 40 micromètres.

Le produit calciné à 550°C dans les mêmes conditions que celles de l'exemple 1, présente un diagramme de diffraction des rayons X analogue à celui du tableau I. Après calcination la teneur en fluor est égale à 0,10 % en poids.

Exemple 4 : Préparation d'une ferrizéosilite suivant l'invention de rapport molaire $SiO_2/Al_2O_3$ égal à 46.

On prépare un mélange ayant la composition molaire suivante : 1 $SiO_2$ (Aerosil) ; 0,05 $FeCl_3$ ; 0,25 TPABr ; 0,5 $NH_4F$ ; 33 $H_2O$ ; en utilisant 8 % des quantités molaires indiquées.

Le mélange (pH = 6) est chauffé pendant 7 jours à 190°C dans le même type d'autoclave que celui de l'exemple précédent ; le pH final est de 6. L'analyse chimique du solide obtenu après sonication (4 g) conduit à un rapport molaire $SiO_2/Fe_2O_3$ égal à 46. La teneur en fluor et de 0,14 % en poids. Le diagramme de diffraction des rayons X du produit calciné à l'air à une température de 550°C pendant 9 heures sous air pur correspond à celui du tableau I.

Les cristaux prismatiques, maclés, ont une taille moyenne de 100 x 50 micromètres.

EXEMPLE 5 : Préparation d'une ferrizéosilite suivant l'invention à partir d'une source de fer et de silice autre que celles utilisées dans l'exemple 1.

Cet exemple illustre la possibilité de partir d'un gel ferrisilicaté comme source de silicium et de fer.

On prépare un gel de composition : 1 $SiO_2$ ; 0,025 $FeCl_3$ par addition, sous agitation, d'une solution de chlorure ferrique à une solution de silicate de sodium. Le pH final du mélange (6,5) est obtenu par ajout d'une solution d'acide nitrique concentré. Afin d'éliminer toutes traces de sodium, l'hydrogel est échangé avec une solution concentrée de nitrate d'ammonium. Après plusieurs lavages, le gel est ensuite séché à 80°C pendant 24 heures.

L'analyse chimique de ce dernier conduit à un rapport molaire Si/Fe de 44. Après homogénéisation de cette poudre, on réalise un mélange dont la composition molaire est la suivante : 1 Si ; 0,023 Fe ; 0,08 TPABr ; 0,5 $NH_4F$ ; 40 $H_2O$. On engage 10 % des quantités molaires indiquées dans un autoclave analogue à ceux décrits dans les exemples précédents puis on chauffe l'ensemble à 170°C pendant 15 jours. Le solide ainsi obtenu (m = 4,9 g) est filtré et lavé à l'eau distillée. Après séchage et calcination à 550°C sous air pendant 3 heures, on obtient une férrizéosilite dont le diagramme de diffraction X est conforme au tableau I. La taille des cristaux est voisine de 30x11 micromètres. Le rapport molaire $SiO_2/Fe_2O_3$ déterminé par analyse chimique est égal à 80, et la teneur en fluor après calcination est d'environ 0,08 % en poids.

EXEMPLE 6 : Préparation d'une ferrizéosilite de rapport molaire $SiO_2/Fe_2O_3$ égal à 196 à partir d'un gel silicaté et conduisant à une répartition homogène du fer au sein des cristaux.

Dans cet exemple on prépare également un gel ferrisilicaté avec comme source de silice, l'ester tétraéthylique de l'acide orthosilicique $Si(OC_2H_5)_4$.

25 $cm^3$ de $Si(OC_2H_5)_4$, 0,3 g de $FeCl_3 6H_2O$ et 50 $cm^3$ d'$H_2O$ sont portés à reflux pendant 3 heures. Après précipitation du gel ferrisilicaté, celui-ci est séché à 80°C pendant 2 jours, puis finement broyé. L'analyse chimique de ce dernier conduit aux pourcentages pondéraux suivants : % Si = 39 ; % Fe = 0,75.

On réalise ensuite un mélange dont la composition molaire est la suivante : 1 Si ; 0,01 Fe ; 0,08 TPABr ; 0,5 $NH_4F$ ; 100 $H_2O$. La fraction molaire engagée est de 5 %. Le pH initial est de 6. L'ensemble placé dans un autoclave est porté à 170°C pendant 15 jours. Après réaction (pH final = 6,5), on obtient 3,52 g d'une ferrizéolite dont l'analyse chimique conduit à un rapport molaire $SiO_2/Fe_2O_3$ de 196. L'analyse à la microsonde électronique sur des échantillons préalablement polis, met en évidence une répartition homogène du fer au sein des cristaux de ferrizéosilite. La taille des cristaux ainsi obtenus est voisine de 30 x 20 micromètres. Le diagramme de diffraction des rayons X de cette ferrizéolite calcinée à l'air à 550°C pendant 9 heures, correspond à celui du tableau I. La teneur en fluor après calcination est égale à 0.03 % en poids.

EXEMPLE 7 : Préparation d'une ferrizéosilite selon l'invention de rapport molaire $SiO_2/Fe_2O_3$ égal à 155 à partir de sources de silice et de fer séparées, conduisant à une répartition inhomogène du fer au sein des cristaux.

Les réactifs utilisés dans cet exemple sont les mêmes que ceux utilisés dans l'exemple 1. La composition molaire du mélange ainsi employé est de : $1SiO_2$ ; $0,013FeCl_3$, $6H_2O$ ; $0,5NH_4F$ ; $100H_2O$ ; $0,08TPABr$. pH initial = 6. On engage 5,5 % des proportions molaires.

Le mélange réactionnel, placé dans le même type d'autoclave que celui de l'exemple précédent est porté à une température de 170°C pendant 15 jours. Après réaction (pH final = 6) on obtient 3,6 g d'une ferrizéosilite dont l'analyse chimique conduit à un rapport molaire $SiO_2/Fe_2O_3$ de 155.

L'étude de ce composé à la microsonde électronique met en évidence une hétérogénéité intracristalline. Les cristaux sont constitués d'un coeur riche en fer dont le rapport molaire $SiO_2/Fe_2O_3$ est voisin de 80 et d'une enveloppe riche en silicium avec un rapport molaire $SiO_2/Fe_2O_3$ voisin de 2000.

La taille des cristaux obtenus est voisine de 50 x 40 micromètres. Le diagramme de diffraction des rayons X de cette férrizéosilite calcinée à l'air à 550°C pendant 9 h et réhydratée correspond à celui du tableau I. Après calcination la teneur en fluor est égal à 0,02 % en poids.

EXEMPLE 8 : Préparation d'une ferrizéosilite suivant l'invention en milieu agité.

Cet exemple illustre la possibilité de travailler en milieu agité et ainsi de diminuer considérablement les temps de réaction. On prépare le mélange réactionnel suivant : 1 SiO$_2$ (Aerosil) ; 0,015 FeCl$_3$6H$_2$O ; 0,2 TPABr : 0,4 NH$_4$F ; 100 H$_2$O. La fraction molaire engagée est de 5,5 %. Le pH initial est de 6. L'ensemble placé dans le même type d'autoclave que précédemment est porté en température dans une étuve équipée d'un système d'agitation mécanique assurant le renversement total des autoclaves, et dont la vitesse de rotation peut être modulée. Les caractéristiques de la réaction pour cet exemple sont les suivantes : température = 170° C ; vitesse de rotation = 17 tours/minute ; durée : 3 jours.

Après réaction (pH final = 6), on obtient 3,8 g d'une ferrizéolite dont les cristaux ont une taille moyenne de 40 x 20 micromètres. L'analyse chimique de cette férrizéosilite conduit à un rapport molaire SiO$_2$/Fe$_2$O$_3$ égal à 140.

Le diagramme de diffraction des rayons X de la férrizéosilite préalablement calcinée sous air à 550° C pendant 9 heures, correspond à celui du tableau I. La teneur en fluor déterminée après calcination est égale à 0,032 % en poids.

EXEMPLE 9 : Préparation de ferrizéosilite suivant l'invention à des températures inférieures à 100° C.

Cet exemple illustre la possibilité d'effectuer des synthèses à une température inférieure à 100° C, l'ajout de germes permettant de réduire la durée de cristallisation.

On réalise un mélange dont la composition molaire est la suivante : 1 SiO$_2$ ; 0,055 FeCl$_3$ ; 0,25 TPABr ; 0,5 NH$_4$F ; 100 H$_2$O. La source de silice utilisée est la même que dans l'exemple précédent. Le mélange réactionnel contient également 1 % en poids, par rapport à la quantité de silice engagée, de cristaux d'une férrizéosilite finement broyée. Le pH initial est de 6.2. L'ensemble placé dans un flacon de polypropylène est porté à une température de 80° C pendant 48 jours. Après plusieurs décantations, lavages et filtrations on obtient 53 g d'une ferrizéosilite dont le diagramme de diffraction X est conforme à celui du tableau I. Le solide est ensuite calciné à 550° C dans les mêmes conditions que celles de l'exemple 1. L'analyse chimique révèle un rapport SiO$_2$/Fe$_2$O$_3$ égal à 72 et une teneur en fluor de 0,09 % en poids.

Des essais d'adsorption du n-hexane et du triméthylpentane ont été effectués sur cet échantillon. Pour une température de 20° C et sous une pression relative de l'adsorbat P/Po = 0,1 ; les quantités adsorbées sont respectivement de 12,1 % et de 8,7 % en poids. Ces caractéristiques sont sensiblement analogues à celles d'une zéolithe du type aluminosilicate.

EXEMPLE 10 (comparatif) : Importance de la présence d'ions fluorure dans le milieu de synthèse.

De manière à déterminer dans quelle mesure la présence d'ions fluorure dans le milieu réactionnel est importante pour la synthèse de ferrizéosilite, on réalise les essais suivants :
.  Essai 1 bis : On réalise une synthèse dans les mêmes conditions que celles de l'exemple 1. La seule différence consiste en ce que l'on n'ajoute pas de fluorure d'ammonium.
.  Essai 2 bis : On réalise une synthèse dans les mêmes conditions que celles de l'exemple 2. La seule différence consiste en ce que l'on n'ajoute pas d'acide fluorhydrique.

Dans les deux cas, essai 1 bis (chauffage à 170° C pendant 15 jours, voir exemple 1) et essai 2 bis (chauffage à 170° C pendant 12 jours) on obtient un solide amorphe, aucune trace de ferrizéosilite n'étant détectée dans la phase solide recueillie.

On a recommencé les essais 1 bis et 2 bis en faisant varier le temps de réaction de 1 jour à 30 jours ; dans aucun cas, de la ferrizéosilite ne s'est formée.

EXEMPLE 11 : Application en conversion du méthanol de ferrizéosilite suivant l'invention.

On a étudié les propriétés catalytiques de la ferrizéosilite obtenue dans l'exemple 9 vis à vis de deux réactions qui sont la conversion du méthanol en hydrocarbures, et l'alkylation du toluène en xylènes. D'une manière générale, le mode opératoire est le suivant. On place dans un réacteur 10 g de ferrizéolite préparée selon l'exemple 9 qui est calcinée et activée sous courant d'azote pendant 16 heures à 400° C. On fait passer, suivant la réaction étudiée, soit du méthanol pur, soit un mélange de méthanol et de toluène (rapport molaire toluène/méthanol = 3,96) sur le catalyseur.

On effectue l'analyse de l'effluent sortant du réacteur pour différents temps de réaction par chromatographie en phase gazeuse.

Les températures de réaction ainsi que les vitesses spatiales horaires (masse de réactif par masse de catalyseur et par heure) pour les deux réactions sont respectivement de 370° C ; 4,3 heures⁻¹ (conversion du méthanol) et de 400° C ; 6,1 heures⁻¹ (alkylation du toluène).

Dans le tableau II ont été reportés les résultats obtenus en conversion du méthanol en hydrocarbures avec la ferrizéosilite de la présente invention et une zéolithe de structure MFI de rapport Si/Fe = 25 préparée en milieu conventionnel (basique) suivant les techniques de l'art antérieur, décrites dans le brevet allemand DE 2831611, les brevets européens EP 813532, EP 884422 et EP 115031.

Dans le tableau III on a reporté les résultats obtenus pour l'alkylation du toluène par le méthanol pour la ferrizéosilite de la présente invention et une zéolithe de structure MFI de rapport Si/Fe = 29 préparée en milieu conventionnel (basique) selon les techniques de l'art antérieur, décrites dans les brevets ci-dessus.

## TABLEAU II.

| | | CONVERSION DU METHANOL ET COMPOSITION DES PRODUITS | | | |
|---|---|---|---|---|---|
| FERRIZÉOSILITE SELON L'INVENTION | Temps (mn) | 30 | 240 | 660 | 2010 |
| | % méthanol | 24 | 29,5 | 43 | 47 |
| | % diméthyléther | 20 | 24,5 | 46 | 42 |
| | % hydrocarbures | 56 | 46 | 11 | 11 |
| | % $C_1$, $C_2$, $C_3$ | 43 | 43 | 41 | 30 |
| | % $C_4 \longrightarrow C_{10}$ | 57 | 57 | 59 | 70 |
| ZSM5 AU FER | % méthanol | 30 | 39 | 50 | 60 |
| | % diméthyléther | 25 | 30 | 35 | 38 |
| | % hydrocarbures | 45 | 31 | 15 | 2 |
| | % $C_1$, $C_2$, $C_3$ | 48 | 46 | 40 | 31 |
| | % $C_4 \longrightarrow C_{10}$ | 52 | 54 | 60 | 69 |

## TABLEAU III.

| | ALKYLATION DU TOLUENE | | | |
|---|---|---|---|---|
| **FERRIZÉOSILITE SELON L'INVENTION** | Temps (mn) | 30 | 195 | 280 | 325 |
| | % conversion toluène * | 12,7 | 11,5 | 11,0 | 10,7 |
| | % conversion méthanol | 99,6 | 99,3 | 99,5 | 99,4 |
| | % sélectivité paraxylène | 62,5 | 59,2 | 59,9 | 56,6 |
| | métaxylène | 16,8 | 17,4 | 17,1 | 18,0 |
| | orthoxylène | 20,7 | 23,4 | 23,0 | 25,4 |
| | % xylènes / toluène converti | 91 | 90 | 92 | 92 |
| **ZSM5 AU FER** | % conversion toluène * | 10.0 | 9.8 | 9.0 | 8.7 |
| | % conversion méthanol | 98.0 | 98.6 | 98.3 | 98.3 |
| | % sélectivité paraxylène | 60.0 | 59.0 | 55.1 | 53.3 |
| | métaxylène | 19.1 | 18.8 | 22.0 | 21.7 |
| | orthoxylène | 20.9 | 22.2 | 22.9 | 25.0 |
| | % xylènes / toluène converti | 88.8 | 89.0 | 89.1 | 89.4 |

\* conversion maximale possible du toluène : 25,35 %
(rapport molaire toluène/méthanol = 3,96).

Le tableau III montre que les ferrizéolites préparées selon l'invention ont des performances meilleures tant au niveau sélectivité qu'activité que des zéolithes au fer de structure MFI préparées dans des milieux conventionnels.

EXEMPLE 12 : Application en oligomérisation d'oléfines des férrizéosilite suivant l'invention.

Dans cet exemple on montre que les ferrozéolites présentent de bonnes performances en oligomérisation des oléfines et que le fluor contenu dans le solide après calcination à 550° C permet d'améliorer les performances catalytiques.

On utilise comme produit de départ la ferrizéosilite de l'exemple 1 dont le rapport molaire $SiO_2/Fe_2O_3$ est égal à 84 et dont la teneur en fluor après la calcination à 550° C (décrite dans l'exemple 1) est de 0,06 % en poids. Cette ferrizéosilite est référencée FZ1. On soumet FZ1 à un traitement de défluoration en la traitant dans une solution de $NH_4OH$ 0,25N à 160° C en autoclave. A l'issue de ce traitement le solide est soumis à une calcination à 550° C sous air pendant 3 heures ; il est alors référencé FZ2. La teneur en fluor de FZ2 est très inférieure à celle de FZ1, elle est inférieure à 0,008 % en poids.

Les ferrizéosilites FZ1 et FZ2 ont été testées en oligomérisation du propène. Pour cela 20 g de ferrizéosilite sont placés au milieu d'un réacteur de 130 cm³ de capacité. Le catalyseur est disposé entre deux lits de billes d'alumine alpha inertes vis à vis de la réaction d'oligomérisation. Le propène a ensuite été injecté dans les conditions suivantes :
- température : 330° C
- pression : 4MPa
- débit horaire de propène : 1 kg/1 kg de catalyseur.

Dans ces conditions opératoire, le taux de transformation du propène est de 83 % pour FZ1 et 68 % pour FZ2. Le produit liquide soutiré, après séparation du propène non transformé, présente les caractéristiques suivantes pour FZ1 et FZ2.

|  | FZ1 | FZ2 |
|---|---|---|
| – densité à 20°C | 0,780 | 0,762 |
| – indice de brome | 80 | 93 |
| – distillation ASTM |  |  |
|     Point initial : °C | 75°C | 54°C |
|     10 % vol. : | 138°C | 118°C |
|     30 % vol. : | 160°C | 142°C |
|     50 % vol. : | 201°C | 186°C |
|     70 % vol. : | 242°C | 226°C |
|     90 % vol. : | 285°C | 270°C |
|     95 % vol. : | 298°C | 284°C |
|     Point final °C: | 305°C | 293°C. |

La répartition par nombre d'atomes de carbone, effectuée par spectrométrie de masse est la suivante pour les deux catalyseurs FZ1 et FZ2.

EP 0 273 816 B1

|  | FZ1 | FZ2 |
|---|---|---|
| C6 | 12,0 % | 17,0 % |
| C7 | 4,9 % | 6,5 % |
| C8 | 6,3 % | 6,8 % |
| C9 | 21,0 % | 23,0 % |
| C10 | 5,5 % | 5,5 % |
| C11 | 6,6 % | 6,4 % |
| C12 | 17,9 % | 17,7 % |
| C13 | 4,5 % | 3,9 % |
| C14 | 3,9 % | 3,2 % |
| C15 | 9,7 % | 4,7 % |
| C16 | 3,0 % | 1,8 % |
| C17 | 2,4 % | 1,9 % |
| C18$^+$ | 2,3 % | 1,6 % |
| Total | 100 | 100 |

Les résultats obtenus montrent que les ferrizéosilites selon l'invention sont actives en oligomérisation des oléfines et que la présence de fluor issu du milieu de synthèse permet d'améliorer les performances catalytiques.

EXEMPLE 13 : Application en craquage catalytique de ferrizéosilites selon l'invention.

Dans une première étape on prépare une zéolithe Y échangée aux terres rares.

50 g d'une zéolithe NaY en poudre de rapport Si/Al = 2,5 sont dispersés dans un litre d'une solution normale en $NH_4NO_3$ et agités pendant 1 heure à 100°C. Le solide est ensuite filtré, lavé puis dispersé dans une solution de nitrate de terres rares (mélange de lanthane, cérium, néodyme et praséodyme essentiellement) à pH 5,5 pendant 1 heure à 100°C. Après un second échange identique au précédent dans une solution fraîche de nitrate de terres rares, la zéolithe est filtrée, lavée à l'eau distillée puis séchée 4 heures à 150°C. Le solide obtenu est référence REY.

Dans une deuxième étape on prépare une ferrizéosilite selon la méthode décrite dans l'exemple 5. Cette ferrizéosilite a un rapport molaire $SiO_2/Fe_2O_3$ de 80. Sa teneur en fluor après calcination à 550°C sous air pendant 9 heures est 0,08 % en poids.

Dans une troisième étape on prépare deux catalyseurs A et B.

Le catalyseur A contient uniquement la zéolithe REY.

Le catalyseur B contient un mélange de zéolithe REY et de ferrizéosilite dans les proportions respectives de 80 et 20 % en poids.

Chaque catalyseur est constitué de 80 % en poids de silice amorphe et 20 % en poids de zéolithe (REY ou mélange REY + ferrizéosilite). Le catalyseur B contient donc 4 % poids de ferrozéosilite. Pour faciliter les tests catalytiques et notamment limiter les pertes de charges qui sont liées à l'utilisation de poudres, chacun des constituants (silice amorphe ou zéolithe) est pastillé puis concassé, et la fraction de granulométrie comprise entre 40 et 200 micromètres est récupérée par tamisage.

Les catalyseurs A et B sont testés en conversion d'un distillat sous vide dans les conditions suivantes :

14

- unité en lit fixe,

- quantité de catalyseur = 4,0 g

- rapport pondéral catalyseur/charge = 4,5

- WHSV= 13,3 h$^{-1}$

- temps de réaction = 60 sec. ("time on stream")

- température du réacteur = 480°C

- charge : densité à 15°C = 0,904

           point d'aniline = 79°C

           % poids S      = 1,3

           % poids N    $< 0,1$

           carbone Conradson % poids = 0,32

           Ni + V ppm $< 1$

           ASTMD 1160 $\begin{cases} \text{P.I.} = 202°C \\ 10 \% = 307°C \\ 50 \% = 402°C \\ 90 \% = 510°C \end{cases}$

Les résultats obtenus sont les suivants :

| | CATALYSEUR A | CATALYSEUR B |
|---|---|---|
| Conversion % | 73,2 | 73,1 |
| Rendement essence $C_5^+$ % poids | 50,1 | 48,0 |
| Rendement gaz ($H_2$ + $C_1$ à $C_4$) % poids | 17,8 | 19,8 |
| coke % poids | 5,3 | 5,3 |
| Indice d'octane recherche calculé (NOR) | 89,3 | 90,9 |
| Propène % poids | 4,5 | 5,0 |
| Butènes % poids | 6,1 | 6,9 |
| Essence potentielle (avec une alkylation oléfine -isobutane ) | 23,1 | 25,9 |

Ces résultats montrent que les ferrozéosilites apportent un gain notable en indice d'octane de l'essence (+ 1,6). La diminution du rendement brut en essence (-2) qui est observée est compensée par une production accrue en propène et butènes : avec le catalyseur B la quantité d'essence supplémentaire que l'on peut espérer récupérer (essence potentielle) par alkylation de ces oléfines avec l'isobutane est en effet supérieure d'environ 2,8 points à celle obtenue avec le catalyseur A.

**Revendications**

1. Zéolithe cristalline synthétique du type ferrisilicate caractérisée par :
   a) la formule chimique approchée suivante :

   $M_{2/n}O, Fe_2O_3, xSiO_2$

   où M représente un proton et/ou un cation métallique,
   n est la valence dudit cation,
   x est un nombre compris entre 40 et 1000,
   b) un diagramme de diffraction des rayons X représenté dans le tableau I de la description les symboles pour caractériser l'intensité étant : FF = trés forte, F = forte, mF = moyenne à forte, m = moyenne, mf = moyenne à faible, f = faible, ff = trés faible, et
   c) une teneur en fluor compris entre environ 0,01 % et 1,6 % en poids.

2. Zéolithe selon la revendication 1 caractérisée en ce qu'elle comporte des cristaux dont au moins une dimension est comprise entre environ 0,05 et 500 micromètres.

3. Zéolithe selon l'une quelconque des revendications 1 et 2 caractérisée en ce que les rapports molaires $SiO_2/Fe_2O_3$ sont compris entre 50 et 750.

4. Zéolithe selon l'une des revendications 1 à 3 caractérisée en ce que le fer est réparti d'une manière homogène ou inhomogène au sein des cristaux.

5. Catalyseur comprenant en poids :
   a) de environ 0 % à environ 99.1 % d'une matrice choisie dans le groupe formé par l'alumine, la silice, la magnésie, la zircone, l'oxyde de titane, l'oxyde de bore, une argile, toute combinaison d'au moins deux des composés précités, et/ou une autre zéolithe,
   b) d'environ 0.1 % à environ 100 % d'une zéolithe selon l'une des revendications 1 à 4.

6. Procédé de préparation d'une zéolithe synthétique cristalline selon l'une des revendications 1 à 4, caractérisée en ce que :
   a) on forme un mélange réactionnel ayant un pH inférieur à environ 10 comprenant de l'eau, au moins une source de silice, au moins une source de sel ferrique, au moins une source d'agent mobilisateur contenant des ions fluorure et/ou au moins une source d'agent structurant pouvant fournir des cations organiques,

   ledit mélange ayant une composition, en termes de rapports molaires, comprise dans les intervalles de valeurs suivants :

   | | |
   |---|---|
   | $SiO_2/Fe_2O_3$ | 5 – 2000 |
   | $Fluorure/SiO_2$ | 0,04– 4 |
   | $Cations\ organiques/SiO_2$ | 0,04– 2 |
   | $H_2O/SiO_2$ | 6 – 500 |

   b) on maintient ledit mélange à une température de chauffage au plus égale à environ 250°C jusqu'à qu'on obtienne un composé cristallin, et
   c) on calcine ledit composé à une température supérieure à 400°C.

7. Procédé selon la revendication 6 dans lequel la source d'agent structurant est une source pouvant fournir des cations organiques choisis parmi les cations tétraalkylammonium, trialkylammonium et tétraalkylphosphonium.

8. Procédé selon la revendication 6 dans lequel la source d'agent structurant est une source pouvant

EP 0 273 816 B1

fournir des cations organiques choisis parmi les cations tétrapropylammonium, tripropylammonium et tétrapropylphosphonium.

9. Procédé selon l'une des revendications 6 à 8 dans lequel on prépare ledit mélange à un pH et avec une composition en termes de rapports molaires compris dans les intervalles de valeurs suivants :

| | | |
|---|---|---|
| pH | 4 | – 8 |
| $SiO_2/Fe_2O_3$ | 10 | – 1000 |
| $Fluorure/SiO_2$ | 0,1 | – 1,5 |
| Cation organique/$SiO_2$ | 0,08– | 1 |
| $H_2O/SiO_2$ | 15 | – 350. |

10. Procédé selon l'une des revendications 6 à 9 dans lequel on ajoute au moins un sel complémentaire audit mélange à une concentration définie dans le rapport molaire, sel complémentaire par rapport à la silice compris environ entre 0,1 et 4 et/ou au moins un germe de cristal de la zéolithe préparée selon l'une des revendications 6 et 7 dans le rapport pondéral cristal/silice compris entre environ 0,01 et 0,1.

11. Procédé selon l'une quelconque des revendications 6 à 10 dans lequel on maintient la température de chauffage du mélange réactionnel entre environ 60 et 210° C pendant une durée d'environ 0,5 à 1100 heures.

12. Utilisation d'une zéolithe cristalline synthétique selon l'une des revendications 1 à 4 à titre de catalyseur ou support de catalyseur dans des procédés de conversion du méthanol en hydrocarbures, d'alkylation du toluène par le méthanol, d'oligomérisation des oléfines, d'amélioration de la tenue au froid des gas oils et de craquage catalytique d'une charge d'hydrocarbures.

**Claims**

1. Synthetic crystalline zeolite of the ferrisilicate type characterized by:
   a) the following approximate chemical formula:

   $M_{2/n}O, Fe_2O_3, xSiO_2$

   where M represents a proton and/or a metal cation,
   n is the valency of said cation,
   x is a number comprised between 40 and 1000,
   b) an x-ray diffraction diagram shown in Table I of the accompanying specification, the symbols to characterize the intensity being : FF = very strong, F = strong, mF = moderate to strong, m = moderate, mf = moderate to weak, f = weak, ff = very weak, and
   c) a fluorine content comprised between about 0.01% and 1.6% by weight.

2. Zeolite according to claim 1, comprising crystals of which at least one dimension is comprised between about 0.05 and 500 micrometers.

3. Zeolite according to claim 1, wherein the molar ratios $SiO_2/Fe_2O_3$ are comprised between 50 and 750.

4. Zeolite according to claim 1, wherein the iron is distributed homogeneously or nonhomogeneously within the crystals.

5. Catalyst comprising by weight:
   a) from about 0% to about 99.1% of a matrix selected from the group formed by alumina, silica, magnesia, zirconia, titanium oxide, boron oxide, a clay, any combination of at least two of the aforesaid compounds, and/or another zeolite.

17

b) from about 0.1% to about 100% of a zeolite according to claim 1.

6. Process for the preparation of a crystalline synthetic zeolite according to claim 1, said process comprising:

a) forming a reaction mixture having a pH less than about 10 comprising water, at least one source of silica, at least one source of ferric salt, at least one source of mobilizing agent containing fluoride ions and/or at least one source of templating agent which can provide organic cations,

said mixture having a composition, in terms of molar ratios, comprised within the following ranges of values:

| | | |
|---|---|---|
| $SiO_2/Fe_2O_3$ | 5 | — 2000 |
| $Fluoride/SiO_2$ | 0.04 | — 4 |
| $Organic\ cations/SiO_2$ | 0.04 | — 2 |
| $H_2O/SiO_2$ | 6 | — 500 |

b) keeping said mixture at a temperature of heating at most equal to about 250° C until a crystalline compound is obtained, and

c) calcining said compound at a tenperature above 400° C.

7. Process according to claim 6 wherein the source of templating agent is a source which can provide organic cations selected from among tetraalkylammonium, trialkylammonium and tetraalkylphosphonium cations.

8. Process according to claim 6 in which the source of templating agent is a source which can provide organic cations selected from among the tetrapropylammonium, tripropylammonium and tetrapropylphosphonium cations.

9. Process according to claim 6, wherein said mixture is prepared at a pH and with a composition in terms of molar ratios comprised within the following ranges of values:

| | | |
|---|---|---|
| pH | 4 | — 8 |
| $SiO_2/Fe_2O_3$ | 10 | — 1000 |
| $Fluoride/SiO_2$ | 0.1 | — 1.5 |
| $Organic\ cation/SiO_2$ | 0.08 | — 1 |
| $H_2O/SiO_2$ | 15 | — 350. |

10. Process according to claim 6, wherein at least one salt complementary to said mixture is added at a concentration defined in the molar ratio, a complementary salt with respect to the silica comprised between about 0.1 and 4 and/or at least one crystal seed of the zeolite prepared according to of claim 6 in the ratio by weight crystal/silica comprised between about 0.01 and 0.1.

11. Process according to claim 6, wherein the heating temperature of the reaction mixture is kept between about 60 and 210° C for a period of about 0.5 to 1100 hours.

12. Method of converting methanol into hydrocarbons, of alkylating toluene with methanol, of oligomerising olefines, of improving the low temperature behaviour of gas oils and of catalytic cracking of a charge of hydrocarbons, comprising the use of a synthetic crystalline zeolite according to claim 1 as catalyst or catalyst support.

**Ansprüche**

1. Kristalliner synthetischer Zeolith vom Typ des Ferrisilikats, gekennzeichnet durch folgende Merkmale:
   a) die angenäherde, chemische Formel lautet:

   $M_{2/n}O$, $Fe_2O_3$, $xSiO_2$

   in welcher Formel M ein Proton und/oder ein Metallkation, n die Wertigkeit dieses Kations und x eine Zahl zwischen 40 und 1000 bedeutet,
   b) ein Röntgenstrahlenbrechungsdiagramm, das in der Tabelle I der beiliegenden Beschreibung wiedergegeben ist, wobei folgende Symbole für die Intensität die angegebene Bedeutung besitzen: FF = sehr stark, F = stark, mf = mittelstark, m = mittel, mf = mittel bis schwach, f = schwach, ff = sehr schwach
   c) ein Gehalt an Fluor, zwischen etwa 0,01 und 1,6 Gew.-%.

2. Zeolith nach Anspruch 1,
   dadurch gekennzeichnet,
   daß er aus Kristallen gebildet ist, die zumindest eine Dimension aufweisen, die zwischen etwa 0,05 und 500 μm liegt.

3. Zeolith nach einem der Ansprüche 1 oder 2,
   dadurch gekennzeichnet,
   daß die Molverhältnisse $SiO_2/Fe_2O_3$ zwischen 50 und 750 liegen.

4. Zeolith nach mindestens einem der Ansprüche 1 bis 3,
   dadurch gekennzeichnet,
   daß das Eisen entweder homogen oder inhomogen im Innern der Kristalle vorhanden ist.

5. Katalysator
   dadurch gekennzeichnet,
   daß er in Gewichtsprozenten folgende Inhaltsstoffe aufweist:
   a) etwa 0 % bis etwa 99,1 % einer Kernsubstanz (Matrize), die gebildet wird durch mindestens ein Glied der Gruppe Aluminiumoxid, Siliciumdioxid, Magnesiumoxid, Zirkonoxid, Titanoxid, Boroxid, Argil sowie aus allen denkbaren Kombinationen zumindest zweier der genannten Inhaltsstoffe und / oder eines anderen Zeoliths,
   b) ungefähr 0,1 % bis etwa 100 % eines Zeolithen, gemäß mindestens einem der Ansprüche 1 bis 4.

6. Verfahren zur Herstellung eines synthetischen kristallinen Zeoliths, gemäß mindestens einem der Ansprüche 1 bis 4,
   gekennzeichnet durch folgende Verfahrensstufen:
   a) man bildet zumindest ein Reaktionsgemisch, das einen pH-Wert unterhalb von 10 aufweist und, das aus Wasser, zumindest einem Siliciumdioxidlieferanten, zumindest einem Lieferanten für ein Salz des dreiwertigen Eisens, zumindest einem Lieferanten eines Mobilisierungsmittels, das Fluorionen enthält und / oder zumindest einen Lieferanten für ein strukturgebendes Mittel, das in der Lage ist, organische Kationen zu liefern, zusammengestzt ist, daß das genannte Gemisch ausgedrückt in Mol-Verhältnissen, eine Zusammensetzung aufweist, die in den folgenden Intervallen liegt

| | | |
|---|---|---|
| $SiO_2/ Fe_2O_3$ | 5 | – 2000 |
| $Fluorid/SiO_2$ | 0,04 | – 4 |
| $Organische\ Kationen/SiO_2$ | 0,04 | – 2 |
| $H_2O/SiO_2$ | 6 | – 500 |

b) man hält dieses Gemisch so lange auf einer Temperatur von 250° C oder darüber, bis man eine

19

kristalline Verbindung erhalten hat und

c) man kalziniert diese erhaltene Verbindung bei einer Temperatur oberhalb 400° C.

7. Verfahren nach Anspruch 6,
dadurch gekennzeichnet,
daß der Lieferant (= Quelle) für das strukturbildende Mittel ein solcher ist, der in der Lage ist, organische Kationen zur Verfügung zu stellen, die zur folgenden Gruppe gehören: Tetraalkylammonium-, Trialkylammonium- und Tetraalkylphosphonium-Kation.

8. Verfahren nach Anspruch 6,
dadurch gekennzeichnet,
daß der Lieferant (= Quelle) für das strukturbildende Mittel ein solcher ist, der in der Lage ist, organische Kationen zur Verfügung zu stellen, die zur folgenden Gruppe gehören: Tetrapropylammonium-, Tripropylammonium- und Tetrapropylphosphonium-Kation.

9. Verfahren nach einem der Ansprüche 6 bis 8,
dadurch gekennzeichnet,
daß man die genannte Mischung bei einem pH-Wert und unter Bedingungen herstellt, deren molare Verhältnisse den im folgenden angegebenen Intervallwerten entspricht:

$$
\begin{array}{llll}
pH & 4 & - & 8 \\
SiO_2/Fe_2O_3 & 10 & - & 1000 \\
Fluorid/SiO_2 & 0,1 & - & 1,5 \\
Organisches\ Kation/SiO_2 & 0,08 & - & 1 \\
H_2O/SiO_2 & 15 & - & 350
\end{array}
$$

10. Verfahren nach einem der Ansprüche 6 bis 9,
dadurch gekennzeichnet,
daß man zumindest ein Komplementärsalz zur Mischung hinzugibt, bei einer Konzentration gemäß der molaren Verhältnisdefinition, wobei das Komplementärsalz im Verhältnis zum Siliciumdioxid, etwa zwischen 0,1 und 4 liegt und / oder zumindest einen Kristallkeim in Gestalt des Zeoliths, der gemäß der Ansprüche 6 und 7 hergestellt wurde, hinzugibt, wobei das Gewichtsverhältnis Kristall-Siliciumdioxid im allgemeinen zwischen 0,01 und 0,1 liegt.

11. Verfahren nach mindestens einem der Ansprüche 6 bis 10,
dadurch gekennzeichnet,
daß man die Temperatur bei der Erhitzung der Reaktionsmischung innerhalb von 0,5 bis 1100 Stunden bei etwa 60 bis 210° C hält.

12. Verwendung des synthetischen kristallinen Zeolithen, gemäß mindestens einer der Ansprüche 1 bis 4, als Katalysator oder als Katalysatorträger bei Verfahren der Umwandlung von Methanol in Kohlenwasserstoffe, der Alkylierung von Toluol durch Methanol, der Oligomerisation von Olefinen, der Verbesserung des Gehalts an kaltbeständigen Inhaltsstoffen von Gasölen und bei der katalytischen Krackung einer Kohlenwasserstoffcharge.